# EUROPEAN PATENT SPECIFICATION

(11) **EP 1 575 597 B1**
(45) Date of publication and mention of the grant of the patent: **07.01.2009**
(21) Application number: 03799978.6
(22) Date of filing: 18.12.2003
(51) Int. Cl.: A61K 31/57, A61K 31/573, A61K 31/575, A61P 27/02

(54) **USE OF RIMEXOLONE IN THE TREATMENT OF DRY EYE**
VERWENDUNG VON RIMEXOLON ZUR BEHANDLUNG VON AUGENTROCKENHEIT
UTILISATION DU RIMEXOLONE DANS LE TRAITEMENT DE LA SÉCHERESSE OCULAIRE

(30) Priority: 24.12.2002 US 436255 P
(43) Date of publication of application: 21.09.2005
(73) Proprietor: Alcon, Inc., 6331 Hünenberg (CH)
(72) Inventor: YANNI, John, M., Burleson, TX 76028 (US); GAMACHE, Daniel, A., Arlington, TX 76017 (US); MILLER, Steven, T., Arlington, TX 76017 (US); CASTILLO, Ernesto, J., Arlington, TX 76016 (US)
(74) Representative: Best, Michael
(86) International application number: PCT/US2003/040374
(87) International publication number: WO 2004/058273

(56) References cited:
- EP-A- 0 280 797
- WO-A-93/17664
- US-A- 5 401 509
- US-A- 6 153 607

## Description

### Background of the Invention

The present invention is directed to the use of an oculosurface selective glucocorticoid having limited ocular bioavailability for the treatment of dry eye.

Dry eye conditions can be caused by a variety of factors. For example, inflammation of the lacrimal gland and denervation of the cornea can curb tear production, and meibomian gland dysfunction and incomplete lid closure are frequently to blame for rapid tear evaporation. The conditions may also be attributable to systemic health factors (e.g., Sjögren's syndrome, other collagen vascular diseases or allergies), medications (e.g., antihistamines) or environmental factors (e.g., dust or smoke). The following publication may be referred to for further background regarding the diagnosis of dry eye conditions and various prior approaches to treating those conditions: "The Once and Future Treatment of Dry Eye", Review of Optometry Online, (February 15, 2000); "Attacking the Root Causes of Ocular Surface Disease", Review of Optometry Online, (June, 1998); and "Dry Eye Syndrome", The EyeSite, (August, 1999).

Dry eye, or keratoconjunctivitis sicca, is a common ophthalmological disorder that affects a significant proportion of the worldwide population. Some of these individuals suffer from Sjogren's disease. Women of post-menopausal age comprise another segment of the dry eye population. Dry eye may afflict individuals with differing severity. In mild cases, a patient may experience burning, a feeling of dryness, and other symptoms of ocular discomfort. In severe cases, vision may be substantially impaired.

Although dry eye may have a variety of unrelated pathogenic causes, all these share as a common effect the breakdown of the ocular tear film, with dehydration of and subsequent damage to the exposed outer ocular surfaces. There is increasing evidence that inflammation may be an important factor in the pathogenesis of keratoconjunctivitis sicca, such as identification of elevated levels of pro-inflammatory mediators including IL-1 in the conjunctival epithelium in Sjögren's patients

Individuals afflicted with the systemic autoimmune disease known as Sjögrens syndrome typically suffer with severe dry eye. In this disease, inflammation of the lacrimal gland impairs normal secretory processes, resulting in abnormalities in the tear film. Changes to the ocular surface include the production and accumulation of a variety of mediators of inflammation. These pro-inflammatory products may be derived from injured corneal and conjunctival epithelial cells as well as the inflamed lacrimal gland.

The prior therapies for dry eye have included both palliative agents, such as artificial tear formulations, and drugs, such as topical steroids, topical retinoids (e.g., Vitamin A), oral pilocarpine, and topical cyclosporin. In general, the palliative therapies are capable of providing short-term relief from some of the symptoms of dry eye, but frequent application of the palliative products to the eye is required to maintain this relief, since these products generally do not eliminate the physiological sources of the dry eye conditions. The drug therapies that have been proposed in the prior art have had limited success in treating dry eye conditions. The limited efficacy of prior drug therapies has generally been attributable to the inability of the drug to eliminate or reduce the root causes of the dry eye conditions, side effects from the drugs that threaten the overall ocular health of the patient or result in poor patient compliance, or a combination of these factors.

The use of sex hormones and glucocorticoids in the treatment of dry eye has been discussed extensively in prior scientific papers and patent publications. The following publications may be referred to for further background in this regard: Marsh and Pflugfelder, "Topical Nonpreserved Methyprednisolone Therapy for Keratoconjunctivitis Sicca in Sjögren Syndrome", Ophthalmology, Volume 106, number 4, pages 881-816 (April, 1999); U.S. Patent No. Re. 34,578 (Lubkin); U.S. Patent No. 5,620,921 (Sullivan); and U.S. Patent No. 6,153,607 (Pflugfelder, et al.).

It is known that certain glucocorticoids have a greater potential for elevating intraocular pressure ("IOP") than other compounds in this class. For example, it is known that prednisolone, which is a very potent ocular anti-inflammatory agent, has a greater tendency to elevate IOP than fluorometholone, which has moderate ocular anti-inflammatory activity.

It is also known that the risk of IOP elevations associated with the topical ophthalmic use of glucocoticoids increases over time. In other words, the chronic (i.e., long-term) use of these agents increases the risk of significant IOP elevations.

Unlike bacterial infections or acute ocular inflammation associated with physical trauma, which require short-term therapy on the order of a few weeks, dry eye conditions require treatment for extended periods of time, generally several months or more. This chronic use of corticosteroids significantly increases the risk of IOP elevations. Prolonged use of corticosteroids is also known to increase the risk of cataract formation.

It has been suggested that the above-cited problems associated with chronic corticosteriod therapy can be addressed by means of a "pulse" treatment regimen, wherein the patient is only treated with potent corticosteroids (e.g., prednisolone) for relatively short, intermittent periods. (See the 1999 article by Marsh and Pflugfelder, cited above.) However, in view of the practical limits of achieving patient compliance with such a regimen, particularly in elderly patients, a more viable solution to the above-discussed problems is needed. The present invention is directed to satisfying this need via the use of very low concentrations of the oculosurface selective glucocorticoid rimexolone.

The use of rimexolone to treat ophthalmic inflammation is described in U.S. Patent No. 4,686,214 (Boltralik). A commercial product containing 1% rimexolone has been marketed by Alcon Laboratories, Inc. for several years under the name "VEXOL® 1% (Rimexolone) Ophthalmic Suspension".

US-A 6,153,607 is directed to preservative-free compositions containing a corticosteroid in a carrier to treat a dry eye condition. Among the many suitable corticosteroids rimexolone (0.1-1%) is mentioned.

US-A 5,401,509 discloses compositions for the treatment of corneal haze containing a steroid in a concentration between about 0.1 and 4.0 wt.-%. In the long list of steroids, reference is also made of rimexolone.

WO 93/17664 discloses topical ophthalmic compositions containing at least one cellulosic polymer and at least one carboxy vinyl polymer. Among the many possible active ingredients, rimexolone is mentioned.

EP 280 797 discloses compositions for the treatment of ophthalmic inflammatory disorders containing a steroid. A general concentration range of 0.05% to 2.0% by weight is disclosed, one of the steroids is rimexolone.

### Summary of the Invention

The present invention is based on a finding that the glucocorticoid rimexolone is particularly well suited for use in the treatment of dry eye conditions, particularly for chronic therapy (i.e, daily administration for extended periods of time, such as several months or more).

Most glucocorticoids, including rimexolone, are relatively insoluble in water. However, there is no direct correlation between aqueous solubility and the ability of these drugs to penetrate the cornea and become dispersed in intraocular fluids and tissues. Two glucocorticoids that are generally considered to be potent ophthalmic anti-inflammatory agents, prednisolone and dexamethasone, are able to penetrate the cornea to a greater extent than rimexolone, and therefore exhibit a much higher level of intraocular bioavailability, but are also relatively insoluble in water.

The present invention is based on a finding that the limited intraocular bioavailability of rimexolone is a significant advantage in the treatment of dry eye conditions, particularly with respect to chronic therapy. The advantages are twofold. First, as a result of the limited corneal penetration of rimexolone, the risks of elevating IOP, precipitating cataract formation, or causing other significant ocular side effects are reduced substantially. As indicated above, this reduction of risks is particularly important in chronic therapy situations. Second, the fact that rimexolone only penetrates the cornea to a limited extent means that most of the drug remains on the surface of the cornea and sclera. This feature of rimexolone is referred to herein as "oculosurface selective".

The selectivity of rimexolone for remaining on the ocular surface, rather than being dispersed throughout the eye, is a distinct advantage in dry eye patients because the target tissues in such patients (i.e., the tissues that are primarily affected) are present on the ocular surface. As a result of this selectivity for the ocular surface, it has been found that rimexolone is effective in treating dry eye conditions, even at very low concentrations. The effectiveness of rimexolone at such low concentrations further reduces the risks of IOP increases, cataract formation and other potential ocular side effects.

The present invention is also based on the finding that ophthalmic suspensions containing very low concentrations of rimexolone can be formulated as preserved, multi-dose products, rather than as preservative-free unit dose products. The use of preserved, multi-dose products for treating dry eye conditions has been discouraged in the prior art (see, e.g., the 1999 article by Marsh and Pflugfelder, cited above).

The prior art teaching to avoid the use of antimicrobial preservatives in ophthalmic products generally, and particularly ophthalmic glucocorticoid products for treating dry eye, is based on the fact that the antimicrobial agent conventionally used to preserve such products, benzalkonium chloride, has been shown to cause ocular irritation and exacerbate ocular inflammatory conditions, such as dry eye.

The prior art therefore teaches that such antimicrobial preservatives should be completely removed. Since the sterility of the ophthalmic products must be maintained, the removal of the antimicrobial preservatives requires that the products be packaged in a unit dose format, i.e., each dose of sterile solution is packaged in a small, sealed plastic vial. This approach has the advantage of eliminating the antimicrobial preservatives entirely, but also has several drawbacks, such as, a risk of microbial contamination of products, inconvenience, wasteful use of packaging materials and costliness.

The present invention has overcome the above-discussed problems by replacing the conventional preservative benzalkonium chloride ("BAC") with a preservative system that is very mild, relative to BAC, but yet effective in preventing microbial contamination of multi-dose ophthalmic compositions containing rimexolone. The preservative system comprises a buffer system having antimicrobial activity, and preferably also a very low concentration of an antimicrobial agent that does not cause irritation or other discomfort when applied to the eyes of dry eye patients. The preferred antimicrobial agent is polyquaternium-1, at a concentration of five parts per million (i.e., 0.0005 w/v %). It has also been determined that the use of a non-hydrophilic suspending agent, preferably polyvinyl pyrrolidone, is advantageous in order to effectively suspend rimexolone and facilitate the use of such low concentrations of polyquaternium-1.

### Brief Description of the Drawings

Figure 1 is a graphic summary of the TBUT data discussed in Example 2;
Figure 2 is a graphic summary of the corneal protection data discussed in Example 2;
Figure 3 is a graphic summary of the TBUT data discussed in Example 3; and
Figure 4 is a graphic summary of the corneal protection data discussed in Example 3.

### Detailed Description of the Invention

The present invention is based on the finding that rimexolone is effective in treating dry eye conditions at concentrations that are significantly less than the concentration currently utilized for treatment of ophthalmic inflammation. The concentrations of rimexolone utilized in the present invention are less than 0.1 weight/volume percent ("w/v%"), while the concentration currently utilized in VEXOL® is 1%. Thus, there is a difference in concentrations of at least tenfold.

The rimexolone concentration range for the compositions of the present invention is 0.001 to less than 0.1 w/v%, preferably 0.05 to 0.075 w/v%. The most preferred concentration is 0.075%.

The compositions of the present invention contain a buffer system that functions to maintain the pH of the compositions at or near physiologically compatible levels, and to provide a low level of antimicrobial activity. The low-level antimicrobial activity helps to prevent contamination of the compositions by bacteria, fungi or other microorganisms. This function is referred to herein as "antimicrobial preservation".

The level of antimicrobial activity required in order to achieve antimicrobial preservation of multi-dose ophthalmic pharmaceutical products is described in the United States Pharmacopoeia ("USP"), European Pharmacopoeia ("EP") and corresponding preservative efficacy standards in other countries. The buffer systems utilized in the present invention provide sufficient antimicrobial activity to either enable such standards to be satisfied without inclusion of any conventional antimicrobial agents, or to enable the concentration of conventional antimicrobial agent to be reduced significantly. The amount of buffer system required to achieve the above-cited objectives is referred to herein as "an antimicrobial enhancing amount".

The compositions of the present invention have a pH of 6 to 8, most preferably about 7.4. The pH of the compositions can be adjusted at the time of manufacture by adding small amounts of sodium hydroxide and/or hydrochloric acid. However, in order to maintain the pH of the compositions within the above-specified range over extended periods of one to two years or longer, a buffering system is required.

The preferred buffer system is a combination of one or more borate components and one or more polyol components that interact to form borate/polyol complexes. The use of borate/polyol complexes to enhance the antimicrobial activity of ophthalmic compositions is described in United States Patent No. 6,143,799 (Chowhan, et al.).

As used herein, the term "borate" refers to boric acid, salts of boric acid and other pharmaceutically acceptable borates, or combinations thereof, and the term "polyol" refers to any compound having at least one hydroxyl group on each of two adjacent carbons that are not in trans configuration relative to each other. The most suitable borates are: boric acid, sodium borate, potassium borate, calcium borate, magnesium borate, manganese borate, and other such borate salts. The polyols can be linear or circular, substituted or unsubstituted, or mixtures thereof, so long as the resultant borate/polyol complex is water-soluble and pharmaceutically acceptable. Such compounds include sugars, sugar alcohols, sugar acids and uronic acids. Preferred polyols are sugars, sugar alcohols and sugar acids, including: mannitol, glycerin, propylene glycol and sorbitol. Especially preferred polyols are mannitol and glycerin; most preferred is mannitol. The molar ratio of borate to polyol is generally between 1:0.1 and 1:10, and is preferably between 1:0.25 and 1:2.5.

The borate polyol complexes are utilized in the compositions of the present invention in an amount between 0.5 to 6.0 percent by weight (wt %), preferably between 1.0 to 2.5 wt %. The optimum amount, however, will depend upon the complexity of the product, since potential interactions may occur with the other components of a composition. Such optimum amount can be readily determined by one skilled in the formulatory arts.

In another preferred embodiment of the invention, the buffer system comprises a combination of tromethamine and boric acid. The concentration of boric acid will generally range from 0.1 to 1.5 w/v%, preferably 0.6 w/v%. The concentration of tromethamine will generally range from 0.05 to 0.6 w/v%, preferably 0.25 w/v%.

The preservative system utilized in the compositions of the present invention preferably also includes a small amount of an antimicrobial agent that is not irritating to the eyes of dry eye patients when repeatedly applied to the eyes over extended periods of time. This agent is referred to herein as a "non-irritating, ophthalmically acceptable antimicrobial agent." It is utilized in an amount sufficient to further enhance the antimicrobial preservative efficacy of the compositions, relative to the level of antimicrobial activity achieved with the buffer system alone. The preferred antimicrobial agent is polyquaternium-1. The concentration of polyquaternium-1 will generally be from about 0.0001 to 0.001 w/v%, preferably about 0.0005 w/v%. The compositions of the present invention do not contain benzalkonium chloride and therefore may be referred to as "BAC-free".

As indicated above, rimexolone is relatively insoluble in water. The aqueous compositions of the present invention are therefore preferably formulated as suspensions.

Rimexolone is an extremely hydrophobic molecule, and consequently it is difficult to maintain it in a suspended state. If the rimexolone does not remain in suspension, it will form a cake that cannot be resuspended upon shaking of the container. This caking problem frequently occurs when cellulosic agents, such as HPMC or HEC, are utilized to suspend rimexolone. However, it has been found that caking can be avoided by using polyvinyl pyrrolidone ("PVP") as the suspending agent. It is believed that PVP interacts with the surfaces of the rimexolone particles to form a protective layer. This interaction results in a steric stabilization of the particles in the suspension.

It has also been determined that PVP does not adversely affect the antimicrobial activity of polyquaternium-1, which is a polymeric quaternary ammonium compound. This compatibility between PVP and polyquaternium-1 allows a very low concentration of polyquaternium-1 to be utilized in the multi-dose, preserved compositions of the present invention (e.g., preferably 0.0005 w/v %).

Based on the above-described advantages, the use of PVP as a suspending agent is preferred.

The amount of suspending agent required can be readily determined by persons skilled in the art, but will generally be in the range of about 0.1 to 2.0 w/v%.

The compositions of the present invention preferably also contain a nonionic surfactant in an amount 0.01 to 0.2 w/v%. Many nonionic surfactants are known that are acceptable for topical ophthalmic formulations. Such surfactants include: tyloxapol; polyoxyethylene sorbitan esters, such as polysorbate 80, polysorbate 60, and polysorbate 20; polyethoxylated castor oils, such as Cremophore EL; polyethoxylated hydrogenated castor oils, such as HCO-40; and poloxamers. The preferred surfactant is tyloxapol at a concentration in the range of 0.01 to 0.1%, preferably 0.008%.

The compositions of the present invention are formulated to be isotonic, relative to the natural tear fluids. The compositions are therefore formulated to have an osmolality of about 280 to 320 milliosmoles per kilogram water ("mOsm/kg"). If necessary, the compositions may contain ophthalmically acceptable tonicity-adjusting agents, such as sodium chloride, potassium chloride, glycerin, sorbitol or mannitol. The preferred tonicity adjusting agent is sodium chloride, at a concentration in the range of 0.4 to 0.6%, preferably 0.6%.

The present invention is thus directed to the use of the above defined composition for the preparation of a topical ophthalmic composition for treating dry eye conditions by applying said ophthalmic composition to the affected eye. The frequency of the application may vary somewhat depending on the severity of the dry eye conditions being treated, but will generally be from two to four times per day (i.e., 24 hours).

The duration of the therapy may also vary somewhat from patient to patient, but the therapy will generally continue for a period of from several weeks (e.g., six or more weeks) to several months (e.g., six or more months). Due to the limited corneal penetration of rimexolone, and the extremely low concentrations of rimexolone employed in the compositions of the present invention, it is possible to utilize the compositions of the present invention for chronic treatment of dry eye conditions with very little risk of elevating intraocular pressure, suppressing the ocular immune response, or other side effects frequently associated with prior uses of corticosteroids in the eye.

The present invention is further illustrated by the following examples.

### Example 1

The following formulation is representative of the preferred topical ophthalmic compositions of the present invention with the exception of the formulation with a rimexolone concentration of 0.1%, which was prepared and tested for comparative purpose:

| **COMPONENT** | **COMPENDIAL DESIGNATION** | **CONCENTRATION (w/v%)** |
|---|---|---|
| Rimexolone | Non-Compendial | 0.005-0.1 |
| Polyquatemium-1 | Non-Compendial | 0.0005 |
| Boric Acid | NF | 0.6 |
| Povidone K90 | USP | 1.5 |
| Tyloxapol | USP | 0.008 |
| Sodium Chloride | USP | 0.5 |
| Mannitol | USP | 0.2 |
| Tromethamine | USP | 0.25 |
| Sodium Hydroxide And/or Hydrochloric Acid | NF | Adjust pH to 7.4 ± 0.2 |
| | NF | |
| Purified Water | USP | Q.S. 100 |

The above-described formulations may be prepared as described below:

A measured amount of rimexolone is micronized using a portion of the specified amount of tyloxapol as a wetting agent and purified water as vehicle in appropriate ball milling or micronization equipment.

In a separate vessel containing purified water (60 to 80°C), the polyvinyl pyrrolidone is added and dissolved. Boric acid, mannitol, sodium chloride, tromethamine and the remainder of the tyloxapol solution are added and dissolved into this solution. The resulting solution is allowed to cool to room temperature and the pH is adjusted to 7.4 using HCl and/or NaOH. The micronized rimexolone slurry containing drug, tyloxapol and purified water is then added to this solution. The resulting suspension is adjusted to final weight, and is then subjected to steam sterilization using an appropriate sterilizing cycle. The suspension is allowed to cool to room temperature, followed by aseptic addition of the calculated quantity of presterilized polyquarternium-1 solution. The suspension is finally adjusted to specified weight by the addition of purified water.

The preserved, multi-dose formulation described above has been evaluated relative to the ability of the formulation to satisfy the preservative efficacy standards of the United States Pharmacopoeia ("USP") and European Pharmacopoeia ("EP") and similar standards. It has been determined that the formulation satisfies the preservative efficacy requirements of the USP and EP.

### Example 2

The ability of low doses of rimexolone to alleviate dry eye conditions was evaluated. The compositions tested were the same as the formulation described in Example 1 above, with rimexolone concentrations of 0.005%, 0.01%, 0.05 and 0.1% (comparative example), respectively. The experimental procedures are described below.

Dry eye was induced in New Zealand white rabbits (approximately 2kg) by eliciting bilateral inflammation of the lacrimal glands. Tear function was assessed by measuring tear breakup time ("TBUT") daily for three days, following the induction of dry eye. TBUT was determined by instilling 5µl sodium fluorescein into the cul de sac and manually blinking the lids to distribute the fluorescein within the tear film. Under slit lamp observation, the eye was held open and the time to tear film breakup recorded. Efficacy was determined by comparing TBUT relative to pre-inflammation baseline values in drug and vehicle treated animals.

In a separate group of animals, susceptibility to desiccation-induced corneal injury was assessed following the induction of lacrimal gland inflammation. Desiccation was initiated by placing the rabbits in a low humidity environment continuously for up to three days. Corneal injury was assessed by determining the uptake of the vital dye, methylene blue. Under general anesthesia, the ocular surface was bathed in a 1% solution of methylene blue for five minutes and then washed. The animals were euthanized, eyes were excised and an 8mm-diameter section of cornea was isolated and extracted overnight. The concentration of extracted dye was determined spectrophotometrically (A₆₆₀). Protection of the cornea is indicated by a lesser uptake of dye in drug treated animals relative to that in vehicle treated rabbits. For both TBUT and corneal injury determinations, dosing (QID) was initiated 24-hours prior to inducing inflammation and was continued for the duration of the study.

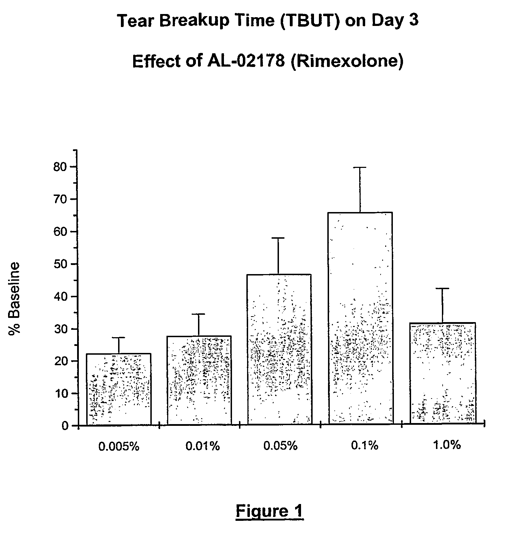

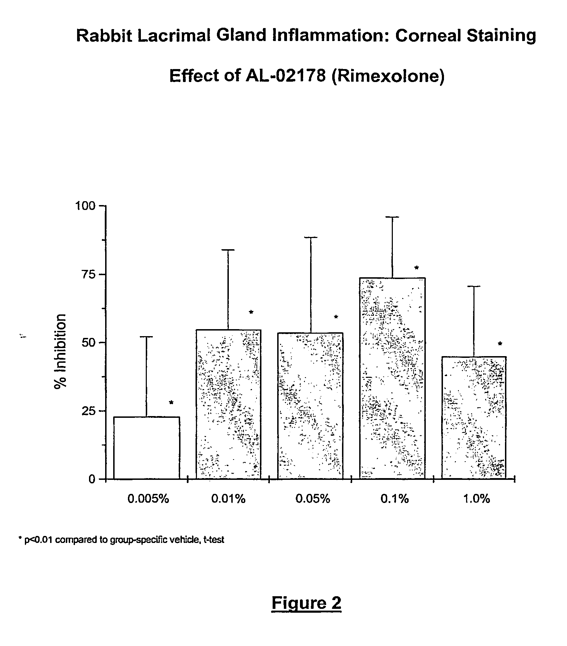

The results of the above-described tests are summarized in Figure 1 and 2, respectively. The low-dose rimexolone formulations of the present invention were significantly effective at each concentration tested. The maximally effective concentration for restoration of TBUT, as well as for prevention of desiccation-induced corneal staining, was 0.1%. Figure 2 illustrates the effect of rimexolone on susceptibility to desiccation-induced corneal injury following lacrimal gland inflammation. A bell-shaped dose-response curve with peak efficacy of 74% inhibition at 0.1% was observed. When TBUT data is expressed as percent of baseline on day three (Figure 1), rimexolone demonstrated a bell-shaped dose-response which was maximal (66% of baseline) at the 0.1% concentration. These results establish that the comparative 0.1% concentration is the maximally effective concentration.

### Example 3

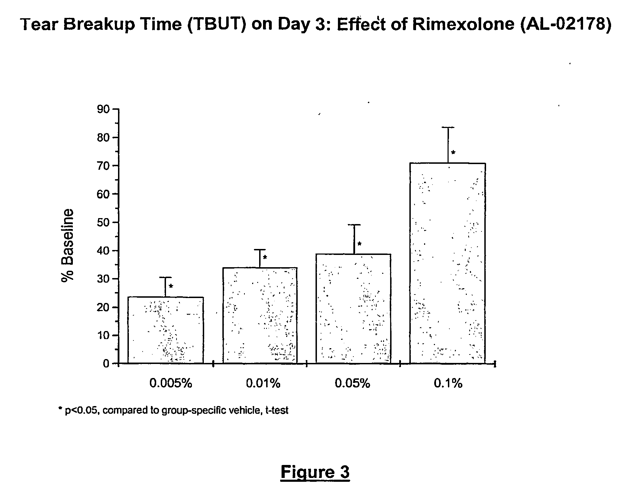

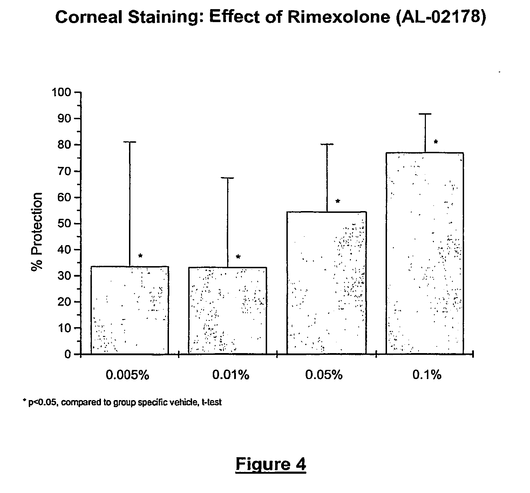

A second dose-response study was conducted using the procedures described in Example 2 above. The results of the study are summarized in Figures 3 and 4.

The rimexolone compositions of Example 1 were significantly effective at each concentration tested. The maximally effective concentration for restoration of TBUT, as well as for prevention of desiccation-induced corneal staining, was 0.1 %. At the comparative 0.1% concentration, rimexolone inhibited corneal staining by 77% (Figure 4) and restored TBUT to 71 % of baseline on day three (Figure 3).

### Example 4

A clinical study in human patients has been conducted to evaluate the efficacy and safety of rimexolone in relieving the ocular signs and symptoms of dry eye in patients with autoimmune connective tissue disease. The formulation described in Example 1 above was utilized in the study. Three different suspensions, containing rimexolone at concentrations of 0.005%, 0.05% and 0.1% (comparative example), respectively, were utilized in the study. A fourth formulation, which was identical to the three rimexolone suspensions except for the elimination of rimexolone, was utilized as a control (i.e., placebo). The number of patients treated with each of the formulations was as follows:

| **Formulation** | **Patients** |
|---|---|
| 0.005% Rimexolone | 30 |
| 0.05% Rimexolone | 26 |
| 0.1 % Rimexolone (comparative example) | 27 |
| Vehicle/Placebo | 23 |

The patients in each group administered one drop of the respective formulations to each eye four times per day for six weeks. Following the end of the six week drug administration period, the patients' symptoms were evaluated.

The results of the study demonstrate that the patients who were treated with the suspensions containing rimexolone had less dry eye symptoms (i.e., discomfort) than the patients treated with the vehicle/placebo. In addition, the patients treated with the rimexolone suspensions did not exhibit any clinically relevant increases in intraocular pressure.

## Claims

1. Use of an oculosurface selective glucocorticoid for the preparation of a topical ophthalmic composition for treating chronic dry eye conditions, by applying a therapeutically effective amount of the topical ophthalmic composition to the cornea of the affected eye on a daily basis for a period of from several weeks to several months or more, wherein the oculosurface selective glucocorticoid comprises rimexolone and wherein the composition contains rimexolone at a concentration of 0.001 to less than 0.1 w/v%.

2. Use according to Claim 1, wherein the composition contains rimexolone at a concentration of 0.05 to 0.075 w/v%.

3. Use according to Claim 1, wherein the composition is a preserved, multi-dose composition.

4. Use according to Claim 3, wherein the composition is an aqueous suspension, and further comprises an amount of polyvinyl pyrrolidone sufficient to suspend the rimexolone in the composition.

5. Use according to Claim 4, wherein the composition further comprises a preservative effective amount of polyquaternium-1.

6. Use according to Claim 5, wherein the composition contains polyquaternium-1 at a concentration of 0.0001 to 0.001 w/v%.

7. Use according to Claim 6, wherein the composition contains polyquaternium-1 at a concentration of 0.0005 w/v%.

8. Use according to Claim 7, wherein the composition further comprises an antimicrobial enhancing amount of a buffer system having antimicrobial activity.

9. Use according to Claim 8, wherein the buffer system comprises a borate/polyol complex.

## Patentansprüche

1. Verwendung eines Augenoberflächen-selektiven Glucocorticoids zur Herstellung einer topischen ophthalmischen Zusammensetzung zur Behandlung chronischer Zustände trockener Augen durch die tägliche Applikation einer therapeutisch wirksamen Menge einer topischen ophthalmischen Zusammensetzung auf die Hornhaut des betroffenen Auges für einen Zeitraum von mehreren Wochen bis mehreren Monaten oder mehr, wobei das Augenoberflächenselektive Glucocorticoid Rimexolon umfaßt und wobei die Zusammensetzung Rimexolon in einer Konzentration von 0,001 bis weniger als 0,1 % Gew./Vol. enthält.

2. Verwendung nach Anspruch 1, wobei die Zusammensetzung Rimexolon in einer Konzentration von 0,05 bis 0,075 % Gew./Vol. enthält.

3. Verwendung nach Anspruch 1, wobei die Zusammensetzung eine konservierte Mehrfachdosenzusammensetzung ist.

4. Verwendung nach Anspruch 3, wobei die Zusammensetzung eine wässerige Suspension ist und ferner eine Menge an Polyvinylpyrrolidon umfaßt, die ausreicht, um das Rimexolon in der Zusammensetzung zu suspendieren.

5. Verwendung nach Anspruch 4, wobei die Zusammensetzung ferner eine konservierende wirksame Menge an Polyquaternium-1 umfaßt.

6. Verwendung nach Anspruch 5, wobei die Zusammensetzung Polyquaternium-1 in einer Konzentration von 0,0001 bis 0,001 % Gew./Vol. enthält.

7. Verwendung nach Anspruch 6, wobei die Zusammensetzung Polyquaternium-1 in einer Konzentration von 0,0005 % Gew./Vol. enthält.

8. Verwendung nach Anspruch 7, wobei die Zusammensetzung ferner eine die antimikrobielle Wirkung steigernde Menge eines Puffersystems mit antimikrobieller Aktivität umfaßt.

9. Verwendung nach Anspruch 8, wobei das Puffersystem einen Borat/Polyol-Komplex umfaßt.

## Revendications

1. Utilisation d'un glucocorticoïde sélectif de surface oculaire pour la préparation d'une composition ophtalmique topique pour le traitement des états chroniques de sécheresse oculaire, par l'application d'une quantité thérapeutiquement efficace de la composition ophtalmique topique à la cornée de l'oeil affecté sur une base journalière pendant une période de plusieurs semaines à plusieurs mois ou plus, dans laquelle le glucocorticoïde sélectif de surface oculaire comprend de la rimexolone et dans laquelle la composition contient de la rimexolone à une concentration de 0,001 à moins de 0,1 % en poids/volume.

2. Utilisation suivant la revendication 1, dans laquelle la composition contient de la rimexolone à une concentration de 0,05 à 0,075 % en poids/volume.

3. Utilisation suivant la revendication 1, dans laquelle la composition est une composition à doses multiples, conservée.

4. Utilisation suivant la revendication 3, dans laquelle la composition est une suspension aqueuse, et de plus comprend une quantité de polyvinyl pyrrolidone suffisante pour mettre en suspension la rimexolone dans la composition.

5. Utilisation suivant la revendication 4, dans laquelle la composition comprend de plus une quantité efficace conservative de polyquaternium-1.

6. Utilisation suivant la revendication 5, dans laquelle la composition contient du polyquaternium-1 à une concentration de 0,0001 à 0,001 % en poids/volume.

7. Utilisation suivant la revendication 6, dans laquelle la composition contient du polyquaternium-1 à une concentration de 0,0005 % en poids/volume.

8. Utilisation suivant la revendication 7, dans laquelle la composition comprend de plus une quantité activatrice antimicrobienne d'un système tampon ayant une activité antimicrobienne.

9. Utilisation suivant la revendication 8, dans laquelle le système tampon comprend un complexe de borate/polyol.
